(19) Europäisches Patentamt
European Patent Office
Office européen des brevets

(11) **EP 3 713 494 B1**

(12) **EUROPEAN PATENT SPECIFICATION**

(45) Date of publication and mention
of the grant of the patent:
**08.03.2023 Bulletin 2023/10**

(21) Application number: **18800196.0**

(22) Date of filing: **15.11.2018**

(51) International Patent Classification (IPC):
**A61B 8/00** *(2006.01)*  **A61B 8/08** *(2006.01)*

(52) Cooperative Patent Classification (CPC):
**A61B 8/469; A61B 8/4263; A61B 8/483;**
A61B 8/463; A61B 8/466; A61B 8/5284; A61B 8/543

(86) International application number:
**PCT/EP2018/081291**

(87) International publication number:
**WO 2019/101609 (31.05.2019 Gazette 2019/22)**

(54) **METHOD AND APPARATUS FOR GUIDING AN ULTRASOUND PROBE**

VERFAHREN UND VORRICHTUNG ZUR FÜHRUNG EINER ULTRASCHALLSONDE

PROCÉDÉ ET APPAREIL PERMETTANT DE GUIDER UNE SONDE À ULTRASONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.11.2017 EP 17306614**

(43) Date of publication of application:
**30.09.2020 Bulletin 2020/40**

(73) Proprietor: **Koninklijke Philips N.V.
5656 AG Eindhoven (NL)**

(72) Inventors:
• **ROUET, Laurence
5656 AE Eindhoven (NL)**
• **DUFOUR, Cecile
5656 AE Eindhoven (NL)**
• **ENTREKIN, Robert Randall
5656 AE Eindhoven (NL)**

• **NG, Gary Cheng-How
5656 AE Eindhoven (NL)**

(74) Representative: **Philips Intellectual Property &
Standards
High Tech Campus 52
5656 AG Eindhoven (NL)**

(56) References cited:
WO-A1-2017/182397    JP-A- 2001 252 268
JP-A- 2014 121 434    US-A1- 2009 024 030
US-A1- 2013 142 010    US-A1- 2015 182 191
US-A1- 2015 366 535

• TREECE G M ET AL: "High-definition freehand 3-D ultrasound", ULTRASOUND IN MEDICINE AND BIOL, NEW YORK, NY, US, vol. 29, no. 4, 1 April 2003 (2003-04-01), pages 529-546, XP004425113, ISSN: 0301-5629, DOI: 10.1016/S0301-5629(02)00735-4

**Description**

FIELD OF THE INVENTION

This invention relates to the field of ultrasound probes, and in particular to the field of guiding an ultrasound probe.

BACKGROUND OF THE INVENTION

Ultrasound imaging is increasingly being employed in a variety of different applications. It is important that the image produced by the ultrasound imaging system is as clear and accurate as possible so as to give the user a realistic interpretation of the subject being scanned. This is especially the case when the subject in question is a patient undergoing a medical ultrasound scan. In this situation, the ability of a clinician to make an accurate diagnosis is dependent on the quality of the image produced by the ultrasound imaging system.

Some ultrasound imaging systems comprise an ultrasound probe and an ultrasound probe tracker, adapted to track a location of the ultrasound probe.

Ultrasound probes may comprise a CMUT transducer array for transmitting ultrasound waves and receiving echo information. The transducer array may alternatively comprise piezoelectric transducers formed of materials such as PZT or PVDF. The transducer array may comprise a two-dimensional array of transducers capable of scanning in a 2D plane or in three dimensions for 3D imaging. In another example, the transducer array may be a 1D array.

Known ultrasound probe trackers include electro-magnetic or optical tracking systems and transducer-based tracking systems.

Methods which generate a three-dimensional (3D) ultrasound image have been proposed. 3D ultrasound images have been shown to significantly improve a clinician's understanding of an imaged volume. To capture a 3D ultrasound image, the ultrasound probe captures a series of ultrasound images and the ultrasound probe tracker identifies a location (of the probe) at which each image is captured. The captured images are stacked, based on their respective locations of capture, to form a 3D image of the imaged volume.

WO 2017/182397 A1 discloses an ultrasound system where a scan pattern is generated (for defining a recommended movement of the probe) based on user registration of anatomical landmarks. An anatomical landmark is registered by placing the probe at a system-prompted position on the subject. US 2015/0366535 A1 discloses a method of tracking a manual imaging probe. An imaging process is tracked to thereby compute whether an imaged image spacing between successive images, captured during an imaging process, exceeds a maximum limit. JP 2014121434 A describes a method of determining whether an ultrasonic probe has deviated from a route along which it should move. US 2013/142010 A1  describes another ultrasound imaging apparatus in which an operator provides information on a direction in which the ultrasound probe is to move. A comparison is made between the actual movement direction and the indicated movement direction, which is presented to a user. US 2009/0024030 A1 describes a method for presenting a suggested path for an ultrasound probe. The suggested path is determined based on a non-ultrasound image, and projected for viewing by a user.

SUMMARY OF THE INVENTION

The invention is defined by the claims. According to examples in accordance with an aspect of the invention, there is provided a method of guiding a user of an ultrasound imaging system during an ultrasound imaging process of a volume, wherein the ultrasound imaging system comprises an ultrasound probe and an ultrasound probe tracker adapted to obtain a location of the ultrasound probe. The method comprises: obtaining a user-defined start location for the ultrasound probe using the ultrasound probe tracker, by determining a location at which the user has positioned the ultrasound probe, to mark the start location, as the start location; obtaining a user-defined end location for the ultrasound probe using the ultrasound probe tracker, by determining a location at which the user has positioned the ultrasound probe, to mark the end location, as the end location, a volume between the start location and the end location thereby defining a volume to be imaged; determining guidance instructions for specifying a movement of the ultrasound probe from the user-defined start location to the user-defined end location, with respect to the volume to be imaged; and during a subsequent ultrasound imaging process of the volume, monitoring, using the ultrasound probe tracker, a movement of the ultrasound probe with respect to the volume to determine whether the user is adhering to the guidance instructions.

It has been recognized that the quality of the ultrasound reconstruction enabled by the ultrasound imaging process (e.g. a 3D ultrasound volume, or a 2D stitch, obtained from the arrangement of a series of 2D ultrasound images) depends heavily upon the experience or training of a user performing the ultrasound imaging process. In particular, poor quality ultrasound images often result from: an ultrasound probe moving at an inappropriate speed and/or over an inappropriate path or failing to provide sufficient ultrasound probe movement to image an entire volume.

There is therefore proposed a method which determines whether a user is following guidance instructions for performing an ultrasound imaging process between a first (start) location and a second (end) location. The first and second locations are defined by the user, such that the user defines a volume or region of interest in which ultrasound imaging is to be performed.

The start location and the end locations are defined before an ultrasound imaging process is performed. Thus, the start and the end locations define a volume or area in which ultrasound imaging is performed. There is therefore presented an a priori methodology for specifying an ultrasound imaging process based on a known volume or area in which ultrasound imaging is to be performed.

The ultrasound imaging process, through which a user is guided, may be a method of capturing ultrasound images and generating a ultrasound output of the volume defined by the start and end locations. The ultrasound output may be a 3D ultrasound image. The guidance instructions may therefore be designed so that the user (following the guidance instructions) generates an improved 3D reconstruction of the volume imaged during the ultrasound imaging process.

The proposed invention advantageously allows a recommended (e.g. optimal) path, speed, angular speed, change in orientation or angle, and/or pattern of movement of the ultrasound probe to be generated before an ultrasound imaging process is performed. In particular, a characteristic of a recommended movement (such as a desired speed or angular speed) is calculated based on a user-defined volume to be imaged. This enables rapid calculation and accurate monitoring of an appropriate movement for the ultrasound probe, without disruption to the ultrasound imaging process.

The invention also ensures an improved success rate of ultrasound imaging, providing higher quality reconstruction of the imaged volume.

The ultrasound probe is a hand-held (portable) probe which can be manually moved over an area of interest.

The user is able to mark the start location and the end location using the (handheld) ultrasound probe to indicate a desired area, region or volume for imaging. The ultrasound probe tracker may detect when the ultrasound probe has been positioned in the start/end location (e.g. held in a particular position for a predetermined period of time, or indicated via a user input such as a button press).

This provides a simple and intuitive mechanism for a user to indicate the start and end locations. Using existing apparatus elements, such as the ultrasound probe tracker, to obtain the user-defined start and end locations reduces a potential complexity to a user and/or an ultrasound imaging system.

By defining a start and end location of the ultrasound imaging process, the method may be adapted for use at non-predetermined areas of a body or subject (i.e. unknown areas of the body). Thus, no prior knowledge of the volume to be image is required in order to generate the guidance instructions for imaging that volume.

The step of determining guidance instructions may comprise: determining a maximum allowable duration of the ultrasound imaging process; determining a distance of a path for the ultrasound probe between the user-defined start location and the user-defined end location; and determining a minimum speed of movement for the ultrasound probe during the ultrasound imaging process based on the maximum allowable duration and the distance.

The guidance instructions may thereby indicate a minimum speed for movement of the ultrasound probe. The minimum speed is calculated based on a distance between the start and the end locations, such as a length of a path for moving the ultrasound probe from the start to the end location, and a maximum allowable duration for an ultrasound imaging process.

The path between the start and end location may be a direct path, a path calculated to optimize a three-dimensional ultrasound image of the volume defined by the user or a sweeping path (i.e. a path repeatedly moving between the start and end locations). In some embodiments, the path represents a change in angle of the ultrasound probe, so that the distance is an angular distance between an angle of the probe at the start location and an angle of the probe at the end location. The path and distance may therefore by linear, non-linear or angular.

Providing a minimum speed as guidance instructions provides an intuitive guidance system for a user which can be easily and readily tracked by the ultrasound probe tracker and by steps according to a method.

The step of monitoring a movement of the ultrasound probe during the ultrasound imaging process may comprise: determining a current location of the ultrasound probe relative to one or more of the user-defined start location and the user-defined end location; determining a current time elapsed during the ultrasound imaging process; determining the average speed of movement during the ultrasound imaging process based on the current location of the ultrasound probe and the current time elapsed; and determining whether the average speed of movement is greater than or equal to the minimum speed of movement to determine whether the user is adhering to the guidance instructions.

Thus, the method may comprise monitoring whether an average speed of the ultrasound probe matches, exceeds or is below the minimum speed of movement for the ultrasound probe. This may provide an indication to a user as to whether they are correctly adhering to the guidance instructions. In particular, the user may be informed as to whether they are to complete the ultrasound imaging process within the maximum allowable time.

This helps prevent an incomplete ultrasound imaging process from being performed, by enabling a user to be prompted to speed up their movement.

In some embodiments, the average speed is instead or further compared to a desired average speed of movement or a historic average speed of movement, such as a speed of movement during a previous ultrasound imaging process. The desired average speed of movement may be determined based on characteristics of the ultrasound imaging process, for example, a 3D ultrasound imaging process may prefer a certain average speed of movement. Maintaining the average speed within a proximity of a desired average speed of movement (or within a desired maximum deviance) may prevent, for example, uneven spatial sampling of the volume.

The maximum allowable duration may be based on one or more of: a user-defined maximum duration; a subject-defined maximum duration; a maximum duration time of an ultrasound imaging process by the ultrasound imaging system; and a frame rate of the ultrasound imaging system and a number of ultrasound images that can be stored by the ultrasound imaging system.

Thus, any one or more characteristics of a subject (upon which an ultrasound imaging process is performed), the user (performing the ultrasound imaging process) and/or the ultrasound imaging system may be used to determine the maximum allowable duration for the ultrasound imaging process.

Basing a maximum allowable duration using a user-defined maximum duration allows a user to control the speed indicated by the guidance instructions. The user-defined maximum duration may change based on, for example, a capability, experience or position of a user.

The maximum allowable duration may be limited, for example, due to a subject condition (such as a maximum breath-hold time). This allows the ultrasound imaging process to be adapted or optimized to suit a subject condition, and prevent unhealthy or dangerous practices by a user for a subject.

Some embodiments use characteristics of the ultrasound imaging system to determine the maximum allowable duration. For example an ultrasound imaging system may be able to store a certain number of ultrasound images or frames. Given a known frame rate, being a rate at which the ultrasound imaging system takes an ultrasound image, a maximum allowable time can be readily calculated. Embodiments may thereby avoid accidental overwriting or incorrect operation of an ultrasound imaging system.

The method may further comprise a step of determining a progress of the ultrasound imaging process based on the monitored movement of the ultrasound probe during the ultrasound imaging process, the user-defined start location and the user-defined end location.

Thus, a progress of the ultrasound imaging process may be determined. This enables an assessment to be made as to whether a user is correctly (i.e. following recommended guidance) or optimally using the ultrasound probe during the ultrasound imaging process.

The method may further comprise a step of displaying the progress of the ultrasound imaging process to a user.

Thus a user may be informed, advised or notified as to their progress with respect to the ultrasound imaging process. In some embodiments, a progress of the ultrasound imaging process is displayed alongside a desired (e.g. optimized) progress of the ultrasound imaging process (e.g. by a certain location or at a certain time), to advise the user whether they are adhering to the recommended/optimized progress of the ultrasound imaging process at any given time.

In some embodiments, the step of determining guidance instructions comprises determining a recommended path of movement of the ultrasound probe.

Thus, the method may comprise recommending a path or trajectory for the ultrasound probe to take when moving from the user-defined start location to the user-defined end location. This may allow for improved or optimized imaging of the volume, by avoiding potentially unnecessary imaging of areas that are not of interest.

The step of monitoring a movement of the ultrasound probe during the ultrasound imaging process may comprise determining a current location of the ultrasound probe; and determining whether the current location is on the recommended path of movement so as to determine whether the user is adhering to the guidance instructions.

In this way, the method may check whether a user is following the recommended path in order to check whether they are adhering or complying with the guidance instructions. In some examples, capturing of images is paused when the user deviates from the recommended path so as to avoid unnecessary capture of redundant images, thereby saving memory space and providing a more efficient system.

According to another aspect of the invention, there is provided an ultrasound imaging method comprising: a method of guiding a user of an ultrasound imaging system as previously described; and an ultrasound imaging process comprising capturing ultrasound images as the ultrasound probe is moved from the user-defined start location to the user-defined end location.

The ultrasound imaging process may also comprise recording a location of the ultrasound probe (using the ultrasound probe tracker) when each ultrasound image is captured.

The ultrasound imaging method may further comprise: determining when the ultrasound probe is located at the user-defined start location and both the user-defined start and end locations have been obtained; and in response to determining that the ultrasound probe is at the user-defined start location and the user-defined start and end locations have been obtained, performing the ultrasound imaging process.

Thus, the method may only start performing an ultrasound imaging process when the ultrasound probe is positioned at the start location. This avoids unnecessary image capture beyond the bounds of the desired volume for imaging (which is defined by the user). This improves a quality and efficiency of an ultrasound image or series of ultrasound images, as unnecessary images are omitted.

The ultrasound imaging method may further comprise generating a three-dimensional ultrasound image of the volume defined by the start location and the end location based on the captured ultrasound images.

Proposed methods are particularly advantageous when used to generate a three-dimensional ultrasound image, as the quality of a three-dimensional ultrasound image is heavily dependent upon a movement of the ultrasound probe between the start and end locations.

In at least one embodiment, the method further comprises obtaining a user-defined intermediate location for the ultrasound probe using the ultrasound probe tracker, wherein the step of determining guidance instructions comprises determining guidance instructions for specifying a movement of the ultrasound probe from the user-defined start location, through the intermediate location and to the user-defined end location, with respect to the volume to be imaged.

There may be provided a computer program comprising code means for implementing any method previously described when said program is run on a computer.

According to an aspect of the invention, there is provided an ultrasound probe guidance system for an ultrasound imaging system having an ultrasound probe and an ultrasound probe tracker adapted to obtain a location of the ultrasound probe. The ultrasound probe guidance system comprises: a volume defining unit adapted to: obtain a user-defined start location for the ultrasound probe using the ultrasound probe tracker, by determining a location at which the user has positioned the ultrasound probe, to mark the start location, as the start location; obtain a user-defined end location for the ultrasound probe using the ultrasound probe tracker, by determining a location at which the user has positioned the ultrasound probe, to mark the end location, as the end location, a volume between the start location and the end location to thereby define a volume in which ultrasound imaging is to be performed; a determining unit adapted to determine guidance instructions for specifying a movement of the ultrasound probe from the user-defined start location to the user-defined end location, with respect to the volume in which ultrasound imaging is to be performed; and a monitoring unit adapted to, during a subsequent ultrasound imaging process, monitor, using the ultrasound probe tracker, a movement of the ultrasound probe with respect to the volume to determine whether the user is adhering to the guidance instructions. The determining unit may be adapted to: determine a maximum allowable duration of the ultrasound imaging process; determine a distance of a path between the user-defined start location and the user-defined end location; and determine a minimum speed of movement for the ultrasound probe during the ultrasound imaging process based on the maximum allowable duration and the distance. The volume defining unit may be further adapted to obtain a user-defined intermediate location for the ultrasound probe using the ultrasound probe tracker; and the determining unit may be adapted to determine guidance instructions by determining guidance instructions for specifying a movement of the ultrasound probe from the user-defined start location, through the intermediate location and to the user-defined end location, with respect to the volume to be imaged.

There is also proposed an ultrasound imaging system comprising: an ultrasound probe; an ultrasound probe tracker; and an ultrasound probe guidance system as previously described.

BRIEF DESCRIPTION OF THE DRAWINGS

Examples of the invention will now be described in detail with reference to the accompanying drawings, in which:

Figure 1 shows a first subject undergoing an ultrasound imaging process;
Figure 2 is a flow chart of a method according to an embodiment;
Figure 3 shows a second subject undergoing an ultrasound imaging process;
Figure 4 conceptually illustrates start and end locations defining a volume of a subject;
Figure 5 illustrates a third subject undergoing an ultrasound imaging process;
Figure 6 conceptually illustrates start and end locations defining a volume of a subject;
Figure 7 illustrates a fourth subject undergoing an ultrasound imaging process;
Figure 8 is a flow-chart of an ultrasound imaging method according to an embodiment; and
Figure 9 illustrates an ultrasound imaging system according to an embodiment.

DETAILED DESCRIPTION OF THE EMBODIMENTS

The invention provides an ultrasound guidance method for an ultrasound imaging system in which user-defined start and end locations define a volume to be imaged. Guidance instructions for an ultrasound process performed on the defined volume may thereby be generated, to help guide the movement of an ultrasound probe during the imaging process. The movement of the ultrasound probe is monitored using an ultrasound probe tracker to ensure that the user is adhering to the guidance instructions.

Embodiments are at least partly based on the realization that a volume to undergo an ultrasound imaging process may be defined before an ultrasound imaging process is performed. In particular, a volume can be defined by obtaining start and end locations for an ultrasound imaging process. Guidance instructions for obtaining an image within this predefined volume may thereby be generated to instruct a user as to how the ultrasound imaging process should be carried out to best advantage (e.g. optimally).

Illustrative embodiments may, for example, be employed in a clinical ultrasound environment. Embodiments are particularly useful when employed in a 3D ultrasound imaging process, where the quality of an image is highly dependent upon capturing characteristics (e.g. speed, number of 2D images, distribution of 2D images and so on) of the ultrasound imaging process.

The invention relates to a guidance system to assist users performing an ultrasound acquisition process using a tracked ultrasound probe. One aim is to help optimize an acquisition speed, pace or directionality in order to reconstruct a good quality (3D) ultrasound image based on an ultrasound imaging of a volume. The guidance system may integrate user and system-based constraints and provide real-time feedback to the user.

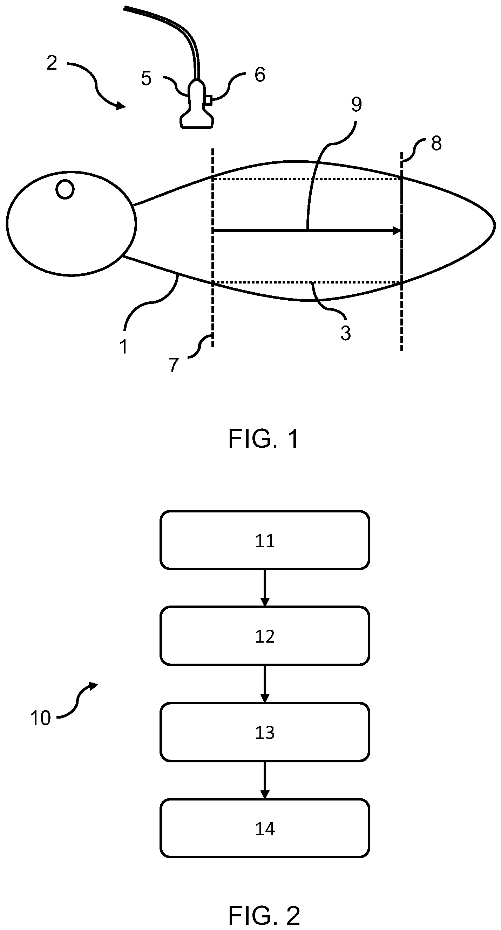

Figure 1 illustrates a first subject 1 undergoing an ultrasound imaging process performed by an ultrasound imaging system 2.

The ultrasound imaging system 2 comprises an ultrasound probe 5 and an ultrasound probe tracker 6. The ultrasound probe 5 comprises a handheld portable probe which is manually movable by a user over an area of interest. The ultrasound probe tracker 6 comprises any known ultrasound probe tracker, such as an electro-magnetic tracked probe.

The ultrasound imaging process, performed by the ultrasound imaging system 2, comprises obtaining, via the ultrasound probe 5, at least one ultrasound image of a volume 3 of the first subject 1. The ultrasound imaging process may further comprise obtaining an ultrasound output of the imaged volume 3, such as a three-dimensional ultrasound image. As will be explained later, the volume 3 is defined by the user of the ultrasound probe 5.

In particular, the ultrasound imaging system captures a plurality of ultrasound images (e.g. two-dimensional ultrasound images) as the ultrasound probe 5 is moved over the subject 1 with respect to the volume 2. These plurality of images may be stacked (based on a positional relationship captured by the ultrasound probe tracker) to generate a three-dimensional image, presented as a series of images or stitched/composited together to generate a larger two-dimensional image.

The present invention recognizes that the quality of a three-dimensional image, or of a stitched 2D image, (re-)constructed from a series of two-dimensional ultrasound images produced by an ultrasound imaging process is affected by characteristics of a movement of the ultrasound probe 5 during the ultrasound imaging process. In particular, the speed, path or pattern of movement affects the quality of an image or series of images.

By way of example, if an ultrasound probe is moved too quickly whilst capturing a plurality of ultrasound images to prepare a three-dimensional ultrasound image, the captured ultrasound images will be too distantly spaced from one another to stack effectively. In particular, an ultrasound imaging process may be unable to accurately interpolate or predict a structure of a volume between two such distantly spaced images. Thus, a generated three-dimensional image may be inaccurate, imprecise or comprise missing information or structural features.

In medical environments, the user of the ultrasound imaging system 2 is typically a clinician performing an ultrasound imaging process on an subject or object, such as a (human or animal) patient. Embodiments are particularly advantageous in a medical context, due to the increased importance of accurate and complete imaging of a volume. The concept of predetermining a volume also enables areas of interest to be predetermined. However, other environments which use ultrasound imaging processes, such as nondestructive testing, are also considered.

The present invention proposes methods of guiding a movement of the ultrasound probe so as to avoid incorrect or non-recommended (e.g. non-optimal) movement of the ultrasound probe during an ultrasound imaging process. Thus, a quality of an output of the ultrasound imaging process may be significantly increased. The ultrasound probe may be moved by a user who performs the imaging, such that guiding the user of an ultrasound imaging system comprises guiding the movement of the ultrasound probe and vice versa.

A method of guiding a user of the ultrasound imaging system 2 is described with reference to both Figure 1 and Figure 2. Figure 2 is a flow chart of a method 10 according to an embodiment.

The method 10 comprises a step 11 of obtaining a user-defined start location 7 for the ultrasound probe 5. The step 11 comprises determining a location 7 at which the ultrasound probe 5 has been positioned. Thus the user marks, using the ultrasound probe 5, a position of the start location 7.

The method 10 also comprises a step 12 of obtaining a user-defined end location 8 for the ultrasound probe 5. Similarly, the step 12 comprises determining a location 8 at which the ultrasound probe 5 has been positioned as the end location. Thus, the user marks, using the ultrasound probe 5, a position of the end location 8.

The start location 7 and the end location 8 thereby together define a volume 3 to be imaged during an ultrasound imaging process. The volume 3 may span between the start location 7 and the end location 8.

The method 10 further comprises a step 13 of determining guidance instructions for specifying a movement of the ultrasound probe with respect to the volume to be imaged. By way of example, the guidance instructions may indicate an optimized, preferred or recommended speed, duration, path 9 and/or pattern of movement of the ultrasound probe during the ultrasound imaging process when moving from the start location to the end location.

The step 13 may determine the recommended movement of the ultrasound probe based on, for example, characteristics of the start/end locations 7, 8, characteristics of the volume 3, characteristics of the ultrasound imaging process and/or characteristics of the ultrasound imaging system 2. Examples will be explained in detail below.

The step 13 of generating guidance instructions may thereby comprise generating guidance instructions based on at least characteristics of the volume, defined by at least the start and end locations, and characteristics or constraints of the ultrasound imaging system.

The optimization may, for example, be any one of the following: maximizing a volume imaged during the ultrasound imaging process (e.g. with respect to a known maximum duration and/or movement restrictions of the ultrasound imaging process); minimizing a movement of the ultrasound probe during the ultrasound imaging process; minimizing a time of the ultrasound imaging process; minimizing a number of images taken during the ultrasound image process (e.g. whilst maintaining accurate imaging); maximizing a number of images taken during the ultrasound image process (e.g. whilst ensuring that the volume is imaged within a known period of time) and minimizing a movement during or a time of the ultrasound imaging process whilst ensuring that more than predetermined portion of the volume is imaged.

The guidance instructions may be displayed, provided or otherwise indicated to the user (e.g. using a display screen, a visual prompt, a printout, an audio indication, audio instructions, haptic feedback and so on). The guidance instructions may be provided prior to and/or during the ultrasound imaging process.

The method 10 further comprises a step 14 of monitoring the movement of the ultrasound probe 5 during the ultrasound imaging process using the ultrasound probe tracker. The method may thereby determine whether the user is adhering to the guidance instructions.

By way of example, if the guidance instructions indicate a preferred speed of movement, the speed of the ultrasound probe may be monitored to determine whether it matches or falls within a predetermined margin or range around the preferred speed of movement. This helps ensure a regular speed of movement, and prevents uneven spatial sampling of the imaged volume. Further examples will be explained in context below.

Preferably, the step 14 of monitoring a movement of the ultrasound probe comprises determining a progress of the ultrasound imaging process, and a progress of the movement of the ultrasound probe during the ultrasound imaging process in particular. This determination may be based on at least the start location, the end location and a current location (or monitored movement) of the ultrasound probe during the ultrasound imaging process.

The progress represents how far through the ultrasound probe is with respect to the movement of the ultrasound probe in the ultrasound imaging process. This may, for example, indicate a predicted distance (of ultrasound probe movement) or time required to complete the movement of the ultrasound probe in the ultrasound imaging process. The progress may be indicated to a user of the ultrasound imaging system using, for example, one or more visual, audio or tactile outputs.

The step 14 preferably comprises determining whether the progress of the ultrasound imaging process matches or is within a predetermined margin around a predicted progress of the ultrasound imaging process. The predetermined range may, for example, be $\pm 1\%$ or $\pm 5\%$ of the predicted progress.

The predicted progress is calculated based on the guidance instructions. In particular, the predicted progress may represent a predicted time/distance remaining to complete the movement of the ultrasound probe in the ultrasound imaging process if the user had adhered to the guidance instructions.

By way of example, if the guidance instructions comprises a recommended speed, it can be readily predicted how far and ultrasound probe should have moved (if adhering to the recommended speed) from the start location or towards the end location at a particular point in time. The predicted distance thereby may represent the predicted progress of the ultrasound probe movement. The predicted distance may be compared to a measured distance of movement, e.g. calculated by determining a distance between a current location and the start location, to determine whether the user is meeting or adhering to the expected or predicted progress of the ultrasound probe.

The adherence of the ultrasound probe to the predicted progress or the guidance instructions may be indicated to a user of the ultrasound imaging system using, for example, one or more visual, audio or tactile outputs, such as a visual alert indicating when a progress has fallen behind a desired or predicted progress or the user fails to adhere to the guidance instructions.

In some embodiments, both a predicted progress of the user (according to the guidance instructions) and an actual progress of the user may be visually displayed to a user, e.g. using respective progress bars. This may allow a user to readily and intuitively understand whether they are adhering to the guidance instructions, as determined by the system, and how they may adjust the movement of the ultrasound probe so as to adhere to the guidance instructions, e.g. speed up movement.

The proposed invention therefore provides a concept of determining guidance instructions for a user-defined volume of a subject, to allow for optimal or improved imaging of the user-defined volume. Use of a user-defined volume allows guidance instructions to be calculated with a high degree of accuracy, as the boundaries of the imaging process may be predetermined (e.g. the size, shape or composition of the volume to be imaged).

The steps 11, 12 of obtaining the start and end locations preferably comprise obtaining the start and/or end locations based on a user action.

For example, the user may mark the start and/or end position by positioning the ultrasound probe 5 in a particular location for a predetermined period of time, or by positioning the ultrasound probe in a location and providing a user input (e.g. pressing a button) to mark said position. Thus, the user may identify start and end locations for the volume. Using a user input provides a greater certainty that a start/end location has been explicitly identified by a user, and may be more intuitive to operate. Using a predetermined period of time increases a simplicity of the ultrasound imaging system, requiring fewer user inputs.

The steps 11, 12 may comprise outputting an audio, visual or tactile output when the start and/or end location has been obtained (e.g. when a user marks the start/end location). For example, the ultrasound probe (held by the user) may emit a beep or a vibration when a start/end location has been successfully marked, recorded or obtained.

Other methods of obtaining the start and/or locations will be apparent to the skilled person, such as marking (e.g. via a user interface) the locations on a simulation of the subject, obtainable from a patient body scan such as a CT or MRI scan, or obtaining historical or default start/end locations (e.g. from previous ultrasound imaging processes).

In a hereafter described first scenario, the determined guidance instructions comprise a recommended (e.g. optimized) speed for movement of the ultrasound probe during an ultrasound imaging process (also called a sweep speed).

One constraint of interest is a maximum time permitted to perform the imaging process. This may be a maximum allowable duration of the ultrasound imaging process. The maximum time or maximum allowable duration may be due to, for example, storage constraints, processing capabilities, procedural efficiencies, recommended procedures or permitted time for imaging (e.g. due to subject limitations, such as limited appointment time).

In particular, the maximum time or maximum allowable duration for performing an ultrasound imaging process may be defined based on (e.g. the lowest value of): a user-defined maximum duration; a subject-defined maximum duration; a maximum duration time of an ultrasound imaging process by the ultrasound imaging system; and a frame rate of the ultrasound imaging system and a number of ultrasound images that can be stored by the ultrasound imaging system.

In one example, if both the image capture rate of the ultrasound imaging system and a storage capacity (i.e. a maximum number of images that can be stored) is known, the maximum time for performing the ultrasound imaging process is readily determinable, by dividing the number of images that can be stored by the image capture rate. The maximum time or duration may vary depending upon a selected image capture rate or an image quality, as high quality images occupy more storage space.

In another example, where the subject is a patient, the maximum allowable duration may be a length of time during which the patient is able to hold their breath or is able to be safely imaged. In this way, the maximum allowable duration may be defined by the subject or user.

The volume may define a distance over which the ultrasound probe is to be moved during an ultrasound imaging process. This distance may be a distance of a predicted path of the ultrasound probe, such as a straight line or natural curved line, a distance of a recommended path 9 (as embodied below) and/or an angular distance. In a simple example, the distance may be defined as a distance of a path 9 directly connecting the start location 7 and the end location 8.

A recommended speed ($v_o$) for the movement of the ultrasound probe may be determined by dividing the distance ($d_v$) defined by the volume 3 by the maximum time ($t_m$) permitted to perform the ultrasound imaging process, such as follows:

$$v_o = \frac{d_v}{t_m} \tag{1}$$

Thus, the recommended speed $v_o$ may represent a minimum speed with which the ultrasound probe can be moved whilst ensuring that the volume is imaged from the user-defined start location to the user-defined end location. This provides an improved imaging of a volume, and thereby improved accuracy of the ultrasound output.

In another example, the recommended speed $v_o$ may be determined based on an image capture rate of the ultrasound imaging system. In some examples, the faster the image capture rate, the faster the ultrasound probe can be moved during an image.

By way of explanation, it is noted that for an ultrasound probe moving at a consistent speed, captured images are more proximate to one another for an ultrasound imaging system having a higher capture rate than a lower capture rate. Thus, ultrasound probes of systems having different capture rates may be moved at different speeds and still capture the same images.

Thus, determining the recommended speed based on the image capture rate provides a more efficient speed for the ultrasound probe, and reduces capture of redundant images. Thus, a time taken to perform the ultrasound imaging process may also be reduced.

The needed separation or distance between different ultrasound images may be determined according to known principles, such as a 3D ultrasound image reconstruction method, desired image resolution, clinician guidelines or user input. The recommended speed $v_o$ (in m/s) may thereby be determined based on a needed separation $d_s$ (in meters) between different ultrasound images and an image capture rate $i_c$ (images per second) of the ultrasound imaging system, for example, as follows:

$$v_o = d_s . i_s \hspace{4cm} (2)$$

The recommended speed $v_o$ may depend upon the desired spatial resolution of the imaged volume. The greater the desired spatial resolution, the lower the recommended speed. Such an embodiment is particularly advantageous for a 3D ultrasound imaging process, as the spatial resolution of a 3D ultrasound image of a volume depends upon the number of 2D images captured within a volume. Thus, the lower the speed of the ultrasound probe, the more images of the volume are captured and the greater the spatial resolution of the generated 3D ultrasound image. There may, for example, be a mapping between a desired spatial resolution and a recommended speed for that resolution, which may vary dependent upon the ultrasound imaging system device. Such an embodiment allows for a more accurate ultrasound imaging process.

In yet another embodiment, the recommended speed $v_o$ may depend upon characteristics of the user or subject. In embodiments, the ultrasound image system and/or ultrasound imaging process may operate according to a gated acquisition method. The recommended speed may be determined based on a gating rate of a gated acquisition process.

The gating rate represents periodic movement of the subject, such as a heartbeat (e.g. cardiac cycle rate), a respiratory movement or a muscle movement. By way of example, the gating rate may indicate a frequency at which a captured image or frame will be disrupted by a periodic movement of a subject. A gating period may represent a number of frames which can be captured or a time period between periodic movements of the subject.

It will be appreciated that captured images for generating the ultrasound output may be selected based on a gating rate, to thereby mitigate against a subject's (involuntary) periodic movements from affecting the ultrasound output.

In a first scenario, selection of the images based on the gating rate is performed as images are being captured. Images may be captured for only a portion of the gating period, for example, to overlap or avoid a periodic movement of the subject. Thus, images may not be continually captured at an image capture rate, but may rather be captured for only a predetermined period of time or number of frames each gating period.

In a second scenario, selection of the images based on the gating rate is performed as images are selected for generating the output. Images may therefore be continually captured during the ultrasound imaging process, but only images which are captured during a predetermined period of time or number of frames each gating period are selected for generating the ultrasound output.

In either event, the recommended speed $v_o$ may be dependent upon the gating rate or gating period. For example, if it is desired for selected or captured images to overlap the periodic movement, the higher the gating rate, the greater the recommended speed $v_o$, as images can be selected at a higher frequency or a smaller distance apart. If it is desired for selected or captured images to avoid the periodic movement, the lower the gating rate, the greater the recommended speed $v_0$, as a greater number of relevant images can be captured between different periodic movements.

Such embodiments improve an accuracy of determining the recommended speed, and improve an accuracy of the ultrasound output of the ultrasound imaging process.

Selecting the recommended speed may use two or more above-described methods, with selection of the lowest (for improved accuracy), highest (for improved efficiency) or average (as a best compromise) of the determined recommended speeds.

The step 14 of monitoring whether the movement of the ultrasound probe adheres to the guidance instructions may determine whether the movement of the ultrasound probe matches or lies within an allowable margin of the determined recommended movement of the ultrasound probe.

By way of example, the ultrasound probe tracker 6 may track a speed of the ultrasound probe 5 to determine whether the actual speed $v_a$ of movement matches or corresponds to the recommended speed $v_o$.

The ultrasound probe tracker 6 may determine whether the current, actual speed, average speed or moving average speed of the ultrasound probe, during an ultrasound imaging process, is within a predetermined range of the recommended speed. The predetermined range may, for example, be $\pm 1\%$ or $\pm 5\%$ of the recommended speed $v_o$. It is preferable to maintain a consistent speed during the ultrasound imaging process, in order to ensure images are captured at regular distances apart. This reduces error in the ultrasound output.

The average speed of the ultrasound probe may be calculated by measuring a length of time since beginning the ultrasound imaging process (from the start location) and a distance between the start location and the current location of the ultrasound probe (using the ultrasound probe tracker). The distance may be divided by the length of time to determine the speed. Other methods of calculating a speed will be readily apparent to the skilled person (e.g. by measuring an amount of movement in a predetermined period of time).

The method 10 may further comprise a step of providing an indication of the adherence of the ultrasound probe to the guidance instruction. This may, for example, be in the form of an audio, visual or tactile output to a user.

For example, a red light may indicate that an (average) speed of the ultrasound probe is too low, a green light may indicate that an (average) speed of the ultrasound probe is within an allowable range and a blue light may indicate that an (average) speed of the ultrasound probe is too high. Other embodiments of providing an indication to the user will be readily apparent to the skilled person.

By prompting a user to move the ultrasound probe at the recommended speed, and alerting the user to a failure to meet the recommended speed, an uneven spatial sampling of the volume can be avoided. This significantly improves an image quality and provides an even spatial separation of the ultrasound images.

In other examples, the image capture process may be paused whilst the ultrasound probe does not adhere to the guidance instructions so as to prevent unnecessary or non-useful ultrasound images from being captured, thereby providing a more efficient system.

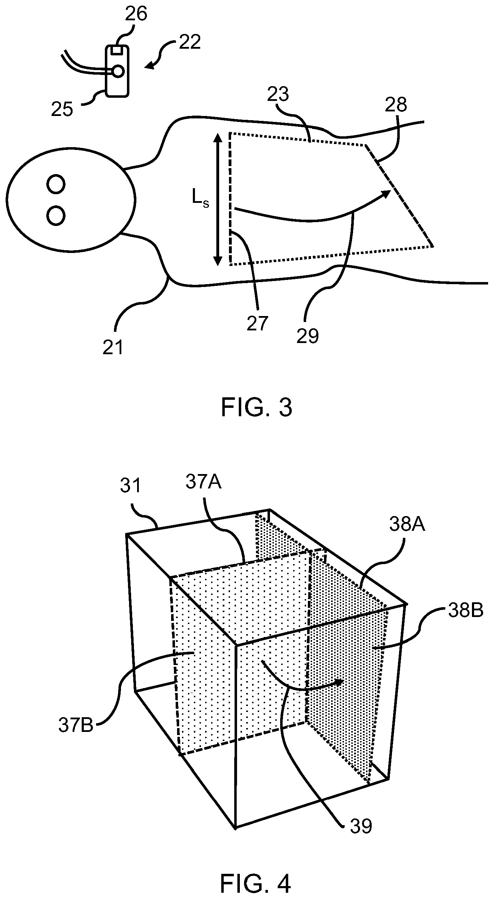

In a hereafter described second scenario, which will be described with reference to Figure 3, the determined guidance instructions comprise a recommended path of movement of the ultrasound probe 5 during an ultrasound image process.

Figure 3 illustrates a plan view of second subject 21 undergoing an ultrasound imaging process performed by an ultrasound imaging system 22. The ultrasound imaging system comprises an ultrasound probe 25 and an ultrasound probe tracker 26, which may be embodied as previously described.

There is defined a volume 23 to be imaged between a start location 27 and an end location 28. Each location may be associated with a respective plane and/or direction. That is, each location may indicate a face or boundary of the volume 23 to be imaged during the ultrasound imaging process.

The direction associated with the start/end locations may be defined, for example, by a user twisting the ultrasound probe to a particular direction when the start/end locations are obtained.

The start location 27 may indicate a plane in which a first ultrasound image of the ultrasound process is to be captured, and the end location 28 may indicate a plane in which a final ultrasound image of the ultrasound process is to be captured.

The start location 27 and end location 28 may each be associated with a length, width, height and/or depth. That is, a plane associated with the start/end location may be finite and have particular boundaries. The characteristics of the start/end locations may be defined by the characteristics of the ultrasound imaging system. For example, a length $L_S$ of a plane associated with the start location may represent a surface of the subject 21 which can be captured in an ultrasound image. A depth and/or shape of that plane (into the subject) may be defined by the imaging capturing capabilities of the ultrasound imaging system.

The volume may comprise the space or matter lying between a plane associated with the start location 27 and a plane associated with the end location 28, for example, comprising all matter lying there between. The volume 23 may be regular/cuboidal or irregular/ non-cuboidal.

Thus, the volume 23 may be defined by a location, orientation, angle, size, shape, extent and/or position of a start plane and an end plane. The start plane and the end plane respectively represent a start location and an end location for the ultrasound probe. One or more characteristics of each of the start plane and the end plane are definable by a user.

The step 13 of generating guidance instructions may comprise generating or determining a recommended path 29 for the ultrasound probe, during an ultrasound imaging process.

The recommended path 29 represents a preferred route or planned trajectory to be taken by the ultrasound probe 25 from the start location 27 to the end location 28 during an ultrasound imaging process. One aim of the recommended path may be to provide an efficient, complete or accurate ultrasound imaging of the volume (e.g. with respect to image quality, volume size, overall ultrasound image output size and so on).

The recommended path 29 may thereby represent a route along the surface of the subject to be taken by the ultrasound probe during the ultrasound imaging process. Ultrasound images taken by the ultrasound imaging system as the probe is moved along the surface of the subject to image the volume 23.

In one example, the recommended path 29 may be selected so that a maximal possible amount of volume 23 can be imaged during an ultrasound imaging process. This may take into account an optimized, average or historic speed of movement of the ultrasound probe and/or a maximum time over which images can be captured. In some examples, the recommended path is selected so that the maximal possible amount of the volume can be imaged within a single, movement or sweep of the ultrasound probe 25. In other examples, the recommended path is selected to provide a minimal movement of the ultrasound probe whilst still imaging more than a predetermined portion (e.g. 80%) of the volume

The recommended path may be restricted to have a maximum angle of translational movement (e.g. so that the ultrasound probe makes no sharp or sudden turns during an ultrasound imaging process if following the recommended path). This maximum angle of movement may be around 90° or around 45°. The recommended path may be a route that images the maximum amount of the volume 23 whilst adhering to this restriction.

The recommended path 29 may be non-linear or otherwise recommend a change in angle of the ultrasound probe, for example, if the planes associated with the start/end locations 27, 28 are non-parallel. The recommended path may also take into account an amount of rotation or a difference in orientation between the plane at the start location 27 and the plane at the end location 29.

The recommended path 29 may, in some embodiments, zigzag, meander, turn back on itself, or pass between the start and end locations more than once. This may allow a greater area of the volume to be imaged with a high degree of accuracy.

Other methods of determining a recommended path or planned trajectory for the ultrasound probe between a start location (or plane) and an end location (or plane) will be apparent to the skilled person.

In some embodiments, a visual display of the planned trajectory is provided to a user, to provide an indication of the recommended path. Optionally, the visual display provides a visual overlay of where the probe is expected to be at a certain or current time during image acquisition and/or a current location of the probe, to indicate a progress of the ultrasound imaging process. In addition, the guidance instructions could be used to provide a directional audio/visual guidance to user to guide the user to (return to) the planned trajectory or recommended path. This could, for example, comprise advising the user to go more left, to go more right, to adjust the angle of the ultrasound probe and so on.

Thus, the location of the ultrasound probe may be identified and used to determine whether the user is adhering to the guidance instructions. In particular, a determination may be made as to whether the user is following the recommended path 29.

A more thorough understanding of the concept of a start location 27 and an end location 28 being associable with a particular plane may be understood with reference to Figure 4.

Figure 4 conceptually illustrates a subject 31, in which a start location 37A and end location 38B are defined by a user.

The start location 37A is associated with a start plane 37B. The start plane 37B represents a plane in which a first ultrasound image of the ultrasound imaging process is to be captured. Similarly, the end location 38A is associated with an end plane 38B, which represents a plane in which a last or final ultrasound image of the ultrasound imaging process is to be captured.

If the start/end locations 37A, 37B were obtained using the ultrasound probe, the orientation of the start plane 37B and/or end plane 38B may depend upon an orientation of the ultrasound probe 25 when the start/end planes were obtained. The ultrasound probe tracker 26 may thereby be adapted to determine an orientation of the ultrasound probe.

The start plane 37B and the end plane 38B may be rotated or differently oriented with respect to one another. Thus, the volume (not shown) located between the two planes may be irregular or otherwise non-cuboidal, for example, a portion of a cylinder.

The recommended path 39 for movement of the ultrasound probe may thereby be non-linear. In particular, the recommended path may cause the ultrasound probe to, during a movement in the ultrasound imaging process, rotate from an orientation of the start plane 37B to an orientation of the end plane 38B.

Generally speaking, the recommended path 39 is a recommended or preferred route for the ultrasound probe, which when taken moves an imaging area of the ultrasound probe (i.e. the current plane in which an image is taken by the ultrasound imaging system) from a plane defined by the start location to a plane defined by the end location.

In some embodiments, the guidance instructions comprises information on both a recommended speed and a recommended path for the ultrasound probe during the ultrasound imaging process. The optimal speed of movement may depend upon (e.g. a distance along) the recommended path 29, 39. The recommended speed may thereby vary along the path, for example, at an earlier point in the path the optimal speed may be a first value, and at a later point in the path, the optimal speed may be a second, different value.

This allows for greater configurability of the guidance instructions and may allow for more accurate imaging of, for example, a particular area of interest in the volume to be imaged.

In one embodiment, the optimal speed toward a center of the recommended path may be less than the optimal speed towards an end or start of the recommended path. This may allow for more accurate imaging towards a center portion of the volume (which may represent an area of greater interest). The area of interest may otherwise be defined by the user, for example, indicating or marking a particular region of interest with the ultrasound probe prior to performing the ultrasound imaging process.

As previously identified, the step of monitoring the ultrasound probe during the ultrasound imaging process may comprise determining a progress of the ultrasound probe along the recommended path 29, 39.

In one example, monitoring the ultrasound probe may determine how far the ultrasound probe is along the recommended path (e.g. a relative distance from the start/end location with respect to the recommended path). This distance may represent the current progress of the ultrasound probe.

In a second example, monitoring the ultrasound probe comprises determining a predicted time remaining for completing the ultrasound imaging process. This may be calculated by determining a distance remaining along the recommended path and multiplying by an average speed of the ultrasound probe.

This progress may be displayed or indicated to a user, for example, using any known visual, audio or tactile output (such as a display, speaker or vibration arrangement).

The progress along the recommended path 29 may be compared to a predicted progress of the ultrasound probe. The predicted progress indicates a time/distance remaining to complete the ultrasound imaging process if the user had adhered to the guidance instructions.

In one example, the predicted progress may be calculated by determining how far along the recommended path 29 an ultrasound probe, moving at a recommended speed, should be from the start location 27.

In a second example, the predicted progress is calculated by determining how long an ultrasound probe moving along the predicted path at a recommended speed should have remaining to complete the ultrasound imaging process.

The predicted progress along the recommended path 29 and the (actual) progress along the recommended path may be displayed or indicated to the user using any method previously described.

The predicted progress and the (actual) progress of the ultrasound image process may be compared, to determine whether the user is adhering to the guidance instructions. If the predicted progress is below the (actual) progress an alert may be generated. This may be indicated to the user in the form of a visual, audio or tactile output. In particular, the user may be advised as to how they may meet the guidance instructions (e.g. speed up movement).

In simple embodiments, such as that described with reference to Figure 1, the recommended path may be replaced by a direct or shortest path from the start location to the second location. Thus, the recommended path may, in some embodiments, be a straight line.

This simplifies a calculation of a progress of the ultrasound probe during the monitoring step 14. In particular, a relative distance from the start location or a relative nearness to an end location may represent a progress of the ultrasound probe during the ultrasound imaging process.

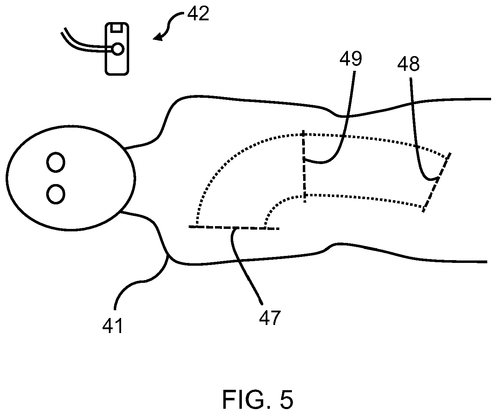

In some embodiments, the volume is defined by more than two locations defined by the user, as will be described with reference to Figure 5, which illustrates a third subject 41 undergoing an ultrasound imaging process performed by an ultrasound imaging system 42.

There is provided a start location 47, and end location 48 and at least one intermediate location 49. The start 47, end 48 and intermediate 49 locations together define the volume to be imaged. For example, the volume may comprise a volume between the start location 47 and the intermediate location 49, together with a volume between the intermediate location 49 and the end location 48.

The location of the intermediate location may thereby define a point on a recommended path between the start location and the end location.

The method may comprise obtaining the intermediate location according to a same concept as described for the start/end locations (e.g. using a user input and/or the ultrasound probe). Thus, the user may mark one or more intermediate locations for the volume.

Such an embodiment may provide a user with a greater amount of control and precision over identifying the volume to be imaged. This advantageously provides a more useful, (clinically) relevant and controllable volume to be imaged.

In some examples, the user may define an entire path of movement for the ultrasound probe during the ultrasound imaging process before the ultrasound imaging process is performed. Put another way, a user may indicate an intended path of movement, for example, by moving the ultrasound probe. This allows a user to rehearse or indicate how they wish the probe to be used prior to an ultrasound imaging process. In some embodiments, determining the guidance instructions may not comprise determining a recommended path, as a desired/intended path has already been defined by the user.

Methods may comprise determining recommended/optimized characteristics for the ultrasound probe when moving along the intended path (e.g. speed or orientation of the ultrasound probe), using any method previously described.

In some embodiments, generating the guidance instructions comprises generating an optimized or recommended pattern of movement of the ultrasound probe. The pattern may be a temporal (e.g. variable speeds of movement) pattern and/or a spatial pattern (e.g. a particular path of movement). The pattern may thereby represent how the ultrasound probe should be moved spatially and/or temporally (e.g. different speeds at different time points) during the ultrasound imaging process.

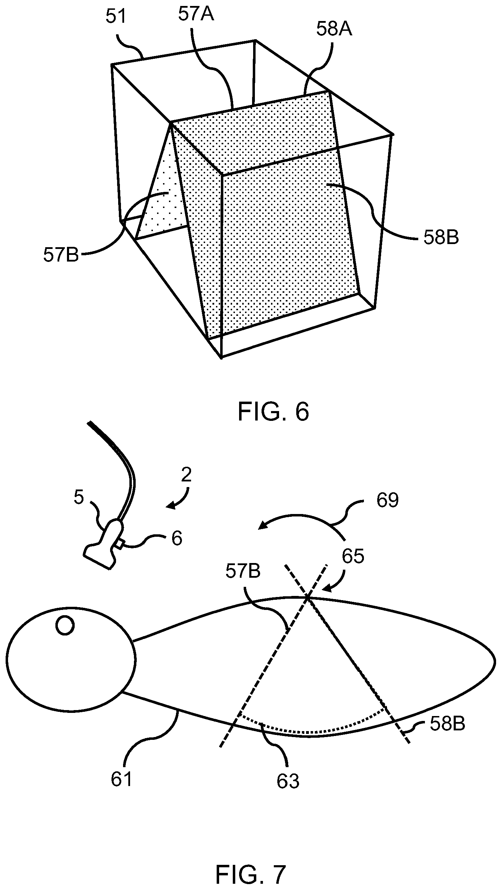

Figures 6 and 7 illustrate a modified embodiment of the invention, in which the start location 57A and the end location 58A are again associated with a start plane 57B and an end plane 58B respectively. Figure 6 conceptually illustrates a subject 51 in accordance with the embodiment, and Figure 7 illustrates the same embodiment in the context of a fourth subject 61 undergoing an ultrasound imaging process.

The start plane 57B and the end plane 58B are angled or inclined with respect to one another. The angle of the start plane 57B and the end plane 58B may be defined by the orientation or inclination of the ultrasound probe (if used to define the start 57A and end 58A locations).

The start and end locations 57A, 58A of the ultrasound probe may be positioned at substantially the same surface location 65 of the subject 61. In such an embodiment, the differing angles of the start and end planes 57B, 58B define the volume 63 to be imaged therebetween.

The guidance instructions comprise instructions for specifying a movement of the ultrasound probe between the start 57B and end planes 58B. In particular, the guidance instructions may comprise a recommended/optimized angular speed of movement between the start and the end planes. The determined guidance instructions may therefore comprise angular guidance instructions.

As the surface location 65 of the start and end locations 57A, 58A may be the same, the guidance instructions may indicate that the translational movement of the ultrasound probe across the surface of the subject 61 remains the same during the ultrasound imaging process.

The guidance instructions may comprise, detail or define a recommended path 69 for the movement of the ultrasound probe 5. The recommended path 69 may therefore indicate an optimal or recommended change in orientation of the ultrasound probe 5, during the ultrasound imaging process, so as to image the volume 63 defined by the first and second angled planes 57B, 58B. Thus, the path 69 may define a recommended change in angle of the ultrasound probe during the ultrasound imaging process.

In one scenario, the ultrasound imaging system may obtain a first angle associated with the first location 57A or first plane 57B and a second angle associated with the second location 58A or second plane 58B. The ultrasound imaging system may determine, e.g. based on previously described characteristics of the ultrasound imaging system, a recommended/optimized angular speed for the ultrasound imaging probe 5 during the ultrasound imaging process. The angular speed may be selected, for example, so as to maximize a number of ultrasound images taken between the start and end location, taking into account a maximum memory size of the ultrasound imaging system. Of course, other previously described methods for calculating a speed of movement of the ultrasound probe may be adapted to calculate an appropriate angular speed of movement.

Of course, the concept of angular guidance may also be incorporated in any previously described guidance methodology, such that the guidance instructions may instruct a change in the surface location, position, orientation, angle and so on of the ultrasound probe during the imaging process. In one scenario, both a surface location and angle of the start and end locations differ.

The guidance instructions may thereby be used to advise a user (e.g. using an audio, visual or tactile output) as to how at least an angular speed or angle of the ultrasound probe, with respect to the subject, should be altered to adhere to a recommended angular speed or angle during the ultrasound imaging process.

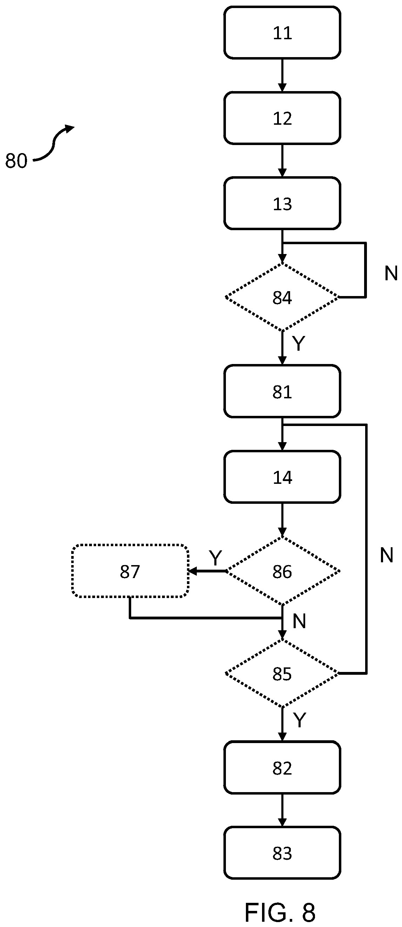

Figure 8 illustrates an ultrasound imaging method 80 according to an embodiment. The ultrasound imaging method 80 comprises a method of guiding a user of an ultrasound imaging system, as previously described, and an ultrasound imaging process.

The guidance method comprises steps of obtaining 11 the user-defined start location, obtaining 12 the user-defined end location, determining 13 guidance instructions for specifying a movement of the ultrasound probe with respect to the volume to be imaged and monitoring 14 a movement of the ultrasound probe with respect to the volume to determine whether the user is adhering to the guidance instructions.

The ultrasound imaging method further comprises an ultrasound imaging process 81, 82.

The ultrasound imaging process comprises a step 81 of starting or beginning a capture process of ultrasound images and a step 82 of ending a capture process of ultrasound images. Between the steps 81 and 82, comprising the capture process, the ultrasound imaging method comprises capturing, obtaining or acquiring ultrasound images of the subject, using the ultrasound probe. Images are captured at an image capture rate of the ultrasound imaging system that performs the capturing of ultrasound images. During capture of an image, a location of that image (as tracked by the ultrasound probe tracker) may also be stored.

The ultrasound imaging method may also comprises a step 83 of generating an ultrasound image output.

Preferably, the step 83 comprises reconstructing a 3D ultrasound image based on ultrasound images captured between steps 81 and 82 of the ultrasound imaging process. Thus, ultrasound images captured during a capture process may be used to reconstruct a 3D ultrasound image of the volume of the subject imaged during the capture process. As previously described, this may be performed by stacking the captured ultrasound images based on the location captured by the ultrasound probe tracker, and optionally interpolating between captured images. Other methods of reconstructing a 3D ultrasound image will be readily apparent to the person skilled in the art.

Generation of a 3D ultrasound image significantly increases a clinical relevance of an imaged volume (in a medical context), but also generally increases an ease and intuitiveness with which a reviewer of the ultrasound output may understand the imaged volume. Described embodiments are particularly advantageous when employed in a 3D ultrasound imaging method, as such imaging methods are particularly susceptible to uneven spatial sampling, incomplete volume coverage and poor spatial resolution. These disadvantages are mitigated by any herein described method.

The step 83 may instead comprise outputting a sequence of 2D ultrasound images or a stitched or composite 2D ultrasound image. Methods of preparing such outputs will be apparent to the skilled person.

The ultrasound imaging method 80 may comprise a step 84 of determining whether the ultrasound probe is at the user-defined start location (e.g. as tracked by the ultrasound probe tracker). The step 84 is performed after the user-defined start location and end location have been obtained.

In response to determining that the ultrasound probe is at the user-defined start location, the method 80 moves to the step 81 of starting a capture process of ultrasound images. In response to determining that the ultrasound probe is not at the user-defined start location, the method performs no action, and repeats the determination of whether the ultrasound probe is at the user-defined start location.

Thus, the ultrasound imaging method 80 may only perform the image capture process when the ultrasound probe is at the user-defined start location. This avoids unnecessary image capture beyond the bounds of the desired volume for imaging (which is defined by the user). This improves a quality of an ultrasound image or series of ultrasound images, as unnecessary images are omitted. Such an embodiment also reduces the capture of redundant images (i.e. outside of the user-defined volume) and thereby provides a more efficient capture system.

In other embodiments, the capture process begins in response to a user input.

The ultrasound imaging method 80 may further comprise a corresponding step 85 of determining whether the ultrasound probe is at the user-defined end location, e.g. as tracked by the ultrasound probe tracker. The step 85 is performed after the step 81 of starting a capture process has been performed.

In response to determining that the ultrasound probe has reached the user-defined end location, the method 80 stops the capture process and performs the step 83 of generating an ultrasound output. In response to determining that the ultrasound probe is not at the user-defined end location, the method performs no action, and continues the image capture process and the determining whether the ultrasound probe is at the user-defined end location.

Thus, the ultrasound imaging method 80 may stop the image capture process when the ultrasound probe has reached the end location. This further prevents images outside of the user-defined volume from being captured, thereby reducing a redundancy and improving an efficiency of the ultrasound imaging process.

In other embodiments, the capture process is ended in response to a user input, or in response to a memory of the ultrasound imaging system filling.

In some embodiments, the method 80 comprises a step 86 of determining whether the user has deviated from the guidance instructions. This may be performed according to any previously described method. In response to determining that the user has deviated from the guidance instructions, the method may comprise a step 87 of alerting the user (e.g. using a visual, audio and/or tactile output) of their deviance. Otherwise, the user is not alerted. The step 87 may otherwise or additionally comprise pausing the image capture process (which may then be resumed when the user or probe re-adheres to the guidance instructions).

There is proposed a processing arrangement or ultrasound guidance system adapted to perform any method previously described. The processing arrangement can be implemented in numerous ways, with software and/or hardware, to perform the various functions required. A processor is one example of a processing arrangement which employs one or more microprocessors that may be programmed using software (e.g., microcode) to perform the required functions. A processing arrangement may however be implemented with or without employing a processor, and also may be implemented as a combination of dedicated hardware to perform some functions and a processor (e.g., one or more programmed microprocessors and associated circuitry) to perform other functions.

Examples of processing arrangement components that may be employed in various embodiments of the present disclosure include, but are not limited to, conventional microprocessors, application specific integrated circuits (ASICs), and field-programmable gate arrays (FPGAs).

In various implementations, a processing arrangement may be associated with one or more storage media such as volatile and non-volatile computer memory such as RAM, PROM, EPROM, and EEPROM. The storage media may be encoded with one or more programs that, when executed on a processing arrangement, perform the required functions. Various storage media may be fixed within a processing arrangement or may be transportable, such that the one or more programs stored thereon can be loaded into a processing arrangement.

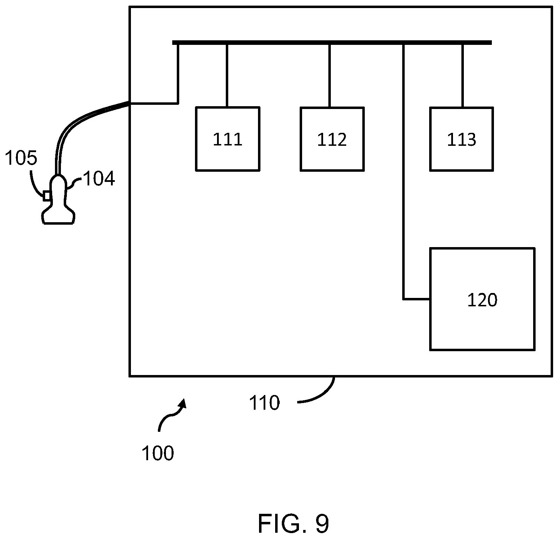

Figure 9 illustrates an ultrasound probe guidance system 110 according to an embodiment in the context of an ultrasound imaging system 100. The overall ultrasound imaging system 100 comprises a (handheld) ultrasound probe 104, an ultrasound probe tracker 105 and the ultrasound probe guidance system 110.

The ultrasound probe guidance system 110 comprises a volume defining unit 111 adapted to obtain a user-defined start location for the ultrasound probe and obtain a user-defined end location for the ultrasound probe, a volume between the start location and the end location thereby defining a volume in which ultrasound imaging is to be performed.

The ultrasound probe guidance system also comprises a determining unit 112 adapted to determine guidance instructions for specifying a movement of the ultrasound probe with respect to the volume in which ultrasound imaging is to be performed.

The ultrasound probe guidance system also comprises a monitoring unit 113 adapted to, during a subsequent ultrasound imaging process, monitor, using the ultrasound probe tracker, a movement of the ultrasound probe with respect to the volume to determine whether the user is adhering to the guidance instructions.

The units 111, 112, 113 of the ultrasound probe guidance system 110 may communicate with one another over a bus, as illustrated, or according to any standard communication protocol. Thus, each unit may be represented by a separate processor. Alternatively, each unit may comprise a functionality of a processor arrangement, such as a specified function or act.

The ultrasound probe guidance system may be adapted to communicate with a user interface 120. The user interface 120 comprises a visual, audio or tactile output. Such outputs may provide any of the previously described outputs. The user interface comprises, for example, a display, a speaker or vibrating means. The ultrasound probe guidance system may be adapted to indicate to a user the guidance information, the progress of the ultrasound probe during the ultrasound imaging process and so on. The user interface 120 may comprise or form the user interface of the overall ultrasound imaging system 100. The user interface may be provided on at least the ultrasound probe (e.g. a vibration means).

The user interface 120 may further comprise a user input, such as one or more buttons or keys. This may allow a user to communicate with the units of the ultrasound probe guidance system. The user input preferably comprises a portion located on the ultrasound probe, for each of access by the user during the ultrasound imaging process.

Whilst embodiments have primarily focused upon determining a recommended speed or path of the ultrasound probe, it will be appreciated that the guidance instructions may indicate other preferred characteristics of the ultrasound probe during the ultrasound imaging process. By way of example, guidance instructions may propose changes to an orientation or angle of the ultrasound probe during an imaging process so as to maximize a volume imaged or to more efficiently image the volume.

Other variations to the disclosed embodiments can be understood and effected by those skilled in the art in practicing the claimed invention, from a study of the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1. A computer implemented method (10) of guiding a user of an ultrasound imaging system (2) during an ultrasound imaging process of a volume (3), wherein the ultrasound imaging system comprises a hand-held ultrasound probe (5) and an ultrasound probe tracker (6) adapted to obtain a location of the ultrasound probe, the method comprising:

   obtaining (11) a user-defined start location (7) for the ultrasound probe using the ultrasound probe tracker, by determining a location (7) at which the user has positioned the ultrasound probe (5), to mark the start location, as the start location;
   obtaining (12) a user-defined end location (8) for the ultrasound probe using the ultrasound probe tracker, by determining a location (8) at which the user has positioned the ultrasound probe (5), to mark the end location, as the end location, a volume between the start location and the end location thereby defining the volume to be imaged;
   determining (13) guidance instructions for specifying a movement of the ultrasound probe from the user-defined start location to the user-defined end location, with respect to the volume to be imaged; and
   during a subsequent ultrasound imaging process of the volume, monitoring, using the ultrasound probe tracker, a movement of the ultrasound probe with respect to the volume to determine whether the user is adhering to the guidance instructions.

2. The method of claim 1, wherein the step of determining guidance instructions comprises:

   determining a maximum allowable duration of the ultrasound imaging process;
   determining a distance of a path for the ultrasound probe between the user-defined start location and the user-defined end location; and
   determining a minimum speed of movement for the ultrasound probe during the ultrasound imaging process based on the maximum allowable duration and the distance.

3. The method of claim 2, wherein the step of monitoring a movement of the ultrasound probe during the ultrasound imaging process comprises:

determining a current location of the ultrasound probe relative to one or more of the user-defined start location and the user-defined end location;

determining a current time elapsed during the ultrasound imaging process;

determining the average speed of movement during the ultrasound imaging process based on the current location of the ultrasound probe and the current time elapsed; and

determining whether the average speed of movement is greater than or equal to the minimum speed of movement to determine whether the user is adhering to the guidance instructions.

4. The method of claim 3, wherein the maximum allowable duration is based on one or more of:

a user-defined maximum duration;

a subject-defined maximum duration;

a maximum duration time of an ultrasound imaging process by the ultrasound imaging system; and

a frame rate of the ultrasound imaging system and a number of ultrasound images that can be stored by the ultrasound imaging system.

5. The method of any of claims 1 to 4, further comprising a step of determining a progress of the ultrasound imaging process based on the monitored movement of the ultrasound probe during the ultrasound imaging process, the user-defined start location and the user-defined end location, optionally wherein the method further comprises a step of displaying the progress of the ultrasound imaging process to a user.

6. The method of any of claims 1 to 5, wherein the step of determining guidance instructions comprises determining a recommended path of movement of the ultrasound probe.

7. The method of claim 6, wherein the step of monitoring a movement of the ultrasound probe during the ultrasound imaging process comprises:

determining a current location of the ultrasound probe; and

determining whether the current location is on the recommended path of movement so as to determine whether the user is adhering to the guidance instructions.

8. An ultrasound imaging method (80) comprising:

a method of guiding a user of an ultrasound imaging system according to any of claims 1 to 7; and

an ultrasound imaging process (81, 82) comprising capturing ultrasound images as the ultrasound probe is moved from the user-defined start location to the user-defined end location.

9. The ultrasound imaging method of claim 8, further comprising:

determining (84) when the ultrasound probe is located at the user-defined start location and both the user-defined start and end locations have been obtained; and

in response to determining that the ultrasound probe is at the user-defined start location and the user-defined start and end locations have been obtained, performing (81) the ultrasound imaging process.

10. The ultrasound imaging method of any preceding claim, further comprising obtaining a user-defined intermediate location (49) for the ultrasound probe using the ultrasound probe tracker,

wherein the step of determining guidance instructions comprises determining guidance instructions for specifying a movement of the ultrasound probe from the user-defined start location (47), through the intermediate location (49) and to the user-defined end location (48), with respect to the volume to be imaged.

11. A computer program comprising code means for implementing the method of any of claims 1 to 10 when said program is run on a computer of an ultrasound imaging system.

12. An ultrasound probe guidance system (110) for an ultrasound imaging system (100) having a handheld ultrasound probe (104) and an ultrasound probe tracker (105) adapted to obtain a location of the ultrasound probe, the ultrasound probe guidance system comprising:

a volume defining unit (111) adapted to:

obtain a user-defined start location for the ultrasound probe using the ultrasound probe tracker, by determining a location (7) at which the user has positioned the ultrasound probe (5), to mark the start location, as the start location;
obtain a user-defined end location for the ultrasound probe using the ultrasound probe tracker, by determining a location (8) at which the user has positioned the ultrasound probe (5), to mark the end location, as the end location, a volume between the start location and the end location to thereby define a volume in which ultrasound imaging is to be performed;

a determining unit (112) adapted to determine guidance instructions for specifying a movement of the ultrasound probe from the user-defined start location to the user-defined end location with respect to the volume in which ultrasound imaging is to be performed; and
a monitoring unit (113) adapted to, during a subsequent ultrasound imaging process, monitor, using the ultrasound probe tracker, a movement of the ultrasound probe with respect to the volume to determine whether the user is adhering to the guidance instructions.

13. The ultrasound probe guidance system of claim 12, wherein the determining unit is adapted to:

determine a maximum allowable duration of the ultrasound imaging process;
determine a distance of a path between the user-defined start location and the user-defined end location; and
determine a minimum speed of movement for the ultrasound probe during the ultrasound imaging process based on the maximum allowable duration and the distance.

14. The ultrasound probe guidance system of claim 12 or 13, wherein:

the volume defining unit is further adapted to obtain a user-defined intermediate location for the ultrasound probe using the ultrasound probe tracker; and
the determining unit is adapted to determine guidance instructions by determining guidance instructions for specifying a movement of the ultrasound probe from the user-defined start location, through the intermediate location and to the user-defined end location, with respect to the volume to be imaged.

15. An ultrasound imaging system (100) comprising:

an ultrasound probe (104);
an ultrasound probe tracker (105);
an ultrasound probe guidance system (110) according to any of claims 11 to 14.


**Patentansprüche**

1. Computerimplementiertes Verfahren (10) zum Führen eines Benutzers eines Ultraschall-Bildgebungssystems (2) während eines Ultraschall-Bildgebungsvorgangs eines Volumens (3), wobei das Ultraschall-Bildgebungssystem eine handgehaltene Ultraschallsonde (5) und einen Ultraschallsonden-Tracker (6) umfasst, der angepasst ist, um eine Position der Ultraschallsonde zu erlangen, das Verfahren umfassend:

Erlangen (11) einer benutzerdefinierten Startposition (7) für die Ultraschallsonde unter Verwendung des Ultraschallsonden-Trackers durch Bestimmen einer Position (7), an der der Benutzer die Ultraschallsonde (5) positioniert hat, um die Startposition zu markieren, als die Startposition;
Erlangen (12) einer benutzerdefinierten Endposition (8) für die Ultraschallsonde unter Verwendung des Ultraschallsonden-Trackers durch Bestimmen einer Position (8), an der der Benutzer die Ultraschallsonde (5) positioniert hat, um die Endposition als die Endposition zu markieren, wobei ein Volumen zwischen der Startposition und der Endposition das abzubildende Volumen definiert;
Bestimmen (13) von Führungsanweisungen zum Spezifizieren einer Bewegung der Ultraschallsonde von der benutzerdefinierten Startposition zu der benutzerdefinierten Endposition in Bezug auf das abzubildende Volumen; und
während eines anschließenden Ultraschall-Bildgebungsvorgangs des Volumens, Überwachung unter Verwendung des Ultraschallsonden-Trackers einer Bewegung der Ultraschallsonde in Bezug auf das Volumen, um zu bestimmen, ob sich der Benutzer an die Führungsanweisungen hält.

2. Verfahren nach Anspruch 1, wobei der Schritt eines Bestimmens von Führungsanweisungen Folgendes umfasst:

Bestimmen einer maximal zulässigen Dauer des Ultraschall-Bildgebungsvorgangs;
Bestimmen eines Abstands eines Wegs für die Ultraschallsonde zwischen der benutzerdefinierten Startposition und der benutzerdefinierten Endposition; und
Bestimmen einer minimalen Bewegungsgeschwindigkeit für die Ultraschallsonde während des Ultraschall-Bildgebungsvorgangs basierend auf der maximal zulässigen Dauer und dem Abstand.

3. Verfahren nach Anspruch 2, wobei der Schritt eines Überwachens einer Bewegung der Ultraschallsonde während des Ultraschall-Bildgebungsvorgangs Folgendes umfasst:

Bestimmen einer aktuellen Position der Ultraschallsonde in Bezug auf eine oder mehrere von der benutzerdefinierten Startposition und der benutzerdefinierten Endposition;
Bestimmen einer aktuellen Zeit, die während des Ultraschall-Bildgebungsvorgangs verstrichen ist;
Bestimmen der durchschnittlichen Bewegungsgeschwindigkeit während des Ultraschall-Bildgebungsvorgangs basierend auf der aktuellen Position der Ultraschallsonde und der aktuell verstrichenen Zeit; und
Bestimmen, ob die durchschnittliche Bewegungsgeschwindigkeit größer als oder gleich wie die Mindestbewegungsgeschwindigkeit ist, um zu bestimmen, ob sich der Benutzer an die Führungsanweisungen hält.

4. Verfahren nach Anspruch 3, wobei die maximal zulässige Dauer auf einem oder mehreren von Folgenden basiert:

einer benutzerdefinierten maximalen Dauer;
einer subjektdefinierten maximalen Dauer;
einer maximalen Dauer eines Ultraschall-Bildgebungsvorgangs durch das Ultraschall-Bildgebungssystem; und
einer Framerate des Ultraschall-Bildgebungssystems und einer Anzahl von Ultraschallbildern, die von dem Ultraschall-Bildgebungssystem gespeichert werden können.

5. Verfahren nach einem der Ansprüche 1 bis 4, ferner umfassend einen Schritt eines Bestimmens eines Fortschritts des Ultraschall-Bildgebungsvorgangs basierend auf der überwachten Bewegung der Ultraschallsonde während des Ultraschall-Bildgebungsvorgangs, der benutzerdefinierten Startposition und der benutzerdefinierten Endposition, wobei das Verfahren optional ferner einen Schritt eines Anzeigens des Fortschritts des Ultraschall-Bildgebungsvorgangs für einen Benutzer umfasst.

6. Verfahren nach einem der Ansprüche 1 bis 5, wobei der Schritt eines Bestimmens von Führungsanweisungen ein Bestimmen eines empfohlenen Bewegungswegs der Ultraschallsonde umfasst.

7. Verfahren nach Anspruch 6, wobei der Schritt eines Überwachens einer Bewegung der Ultraschallsonde während des Ultraschall-Bildgebungsvorgangs Folgendes umfasst:

Bestimmen eine aktuellen Position der Ultraschallsonde; und
bestimmen, ob die aktuelle Position auf dem empfohlenen Bewegungsweg ist, um zu bestimmen, ob sich der Benutzer an die Führungsanweisungen hält.

8. Ultraschall-Bildgebungsverfahren (80), umfassend:

ein Verfahren zum Führen eines Benutzers eines Ultraschall-Bildgebungssystems nach einem der Ansprüche 1 bis 7; und
einen Ultraschall-Bildgebungsvorgang (81, 82), umfassend ein Aufnehmen von Ultraschallbildern, während die Ultraschallsonde von der benutzerdefinierten Startposition zu der benutzerdefinierten Endposition bewegt wird.

9. Ultraschall-Bildgebungsverfahren nach Anspruch 8, ferner umfassend:

Bestimmen (84), wann sich die Ultraschallsonde an der benutzerdefinierten Startposition befindet und sowohl die benutzerdefinierte Start- als auch die Endposition erlangt worden sind; und
als Reaktion auf ein Bestimmen, dass die Ultraschallsonde an der benutzerdefinierten Startposition ist und die benutzerdefinierte Start- und Endposition erlangt wurden, Ausführen (81) des Ultraschall-Bildgebungsvorgangs.

10. Ultraschall-Bildgebungsverfahren nach einem der vorherigen Ansprüche, ferner umfassend ein Bestimmen einer benutzerdefinierten Zwischenposition (49) für die Ultraschallsonde unter Verwendung des Ultraschallsonden-Trackers,

wobei der Schritt eines Bestimmens von Führungsanweisungen ein Bestimmen von Führungsanweisungen zum Spezifizieren einer Bewegung der Ultraschallsonde von der benutzerdefinierten Startposition (47) über die Zwischenposition (49) und zu der benutzerdefinierten Endposition (48) in Bezug auf das abzubildende Volumen umfasst.

11. Computerprogramm, umfassend Codeeinrichtungen zum Implementieren des Verfahrens nach einem der Ansprüche 1 bis 10, wenn das Programm auf einem Computer eines Ultraschall-Bildgebungssystems ausgeführt wird.

12. Ultraschallsonden-Führungssystem (110) für ein Ultraschall-Bildgebungssystem (100), das eine handgehaltene Ultraschallsonde (104) und einen Ultraschallsonden-Tracker (105), der angepasst ist, um eine Position der Ultraschallsonde zu erlangen, aufweist, das Ultraschallsonden-Führungssystem umfassend:
eine Volumendefinitionseinheit (111), die zu Folgendem angepasst ist:

Erlangen einer benutzerdefinierten Startposition für die Ultraschallsonde unter Verwendung des Ultraschallsonden-Trackers durch Bestimmen einer Position (7), an der der Benutzer die Ultraschallsonde (5) positioniert hat, um die Startposition zu markieren, als die Startposition;
Erlangen einer benutzerdefinierten Endposition für die Ultraschallsonde unter Verwendung des Ultraschallsonden-Trackers, durch Bestimmen einer Position (8), an der der Benutzer die Ultraschallsonde (5) positioniert hat, um die Endposition als die Endposition zu markieren, eines Volumens zwischen der Startposition und der Endposition, um dadurch ein Volumen zu definieren, in dem eine Ultraschall-Bildgebung ausgeführt werden soll;
eine Bestimmungseinheit (112), die angepasst ist, um Führungsanweisungen zum Spezifizieren einer Bewegung der Ultraschallsonde von der benutzerdefinierten Startposition zu der benutzerdefinierten Endposition in Bezug auf das Volumen zu bestimmen, in dem eine Ultraschall-Bildgebung ausgeführt werden soll; und
eine Überwachungseinheit (113), die angepasst ist, um während eines nachfolgenden Ultraschall-Bildgebungsvorgangs unter Verwendung des Ultraschallsonden-Trackers eine Bewegung der Ultraschallsonde in Bezug auf das Volumen zu überwachen, um zu bestimmen, ob sich der Benutzer an die Führungsanweisungen hält.

13. Ultraschallsonden-Führungssystem nach Anspruch 12, wobei die Bestimmungseinheit zu Folgendem angepasst ist:

Bestimmen einer maximal zulässigen Dauer des Ultraschall-Bildgebungsvorgangs;
Bestimmen eines Abstands eines Wegs zwischen der benutzerdefinierten Startposition und der benutzerdefinierten Endposition; und
Bestimmen einer minimalen Bewegungsgeschwindigkeit für die Ultraschallsonde während des Ultraschall-Bildgebungsvorgangs basierend auf der maximal zulässigen Dauer und dem Abstand.

14. Ultraschallsonden-Führungssystem nach Anspruch 12 oder 13, wobei:

die Volumendefinitionseinheit ferner angepasst ist, um eine benutzerdefinierte Zwischenposition für die Ultraschallsonde unter Verwendung des Ultraschallsonden-Trackers zu erlangen; und
die Bestimmungseinheit angepasst ist, um durch Bestimmen von Führungsanweisungen zum Spezifizieren einer Bewegung der Ultraschallsonde von der benutzerdefinierten Startposition über die Zwischenposition und zu der benutzerdefinierten Endposition in Bezug auf das abzubildende Volumen Führungsanweisungen zu bestimmen.

15. Ultraschall-Bildgebungsverfahren (100), umfassend:

eine Ultraschallsonde (104);
einen Ultraschallsonden-Tracker (105);
ein Ultraschallsonden-Führungssystem (110) nach einem der Ansprüche 11 bis 14.

**Revendications**

1. Procédé mis en œuvre par ordinateur (10) pour guider un utilisateur d'un système d'imagerie par ultrasons (2) pendant un processus d'imagerie par ultrasons d'un volume (3), dans lequel le système d'imagerie par ultrasons comprend une sonde à ultrasons portative (5) et un dispositif de suivi de sonde à ultrasons (6) adapté pour obtenir un emplacement de la sonde à ultrasons, le procédé consistant à: obtenir (11) un emplacement de départ défini par l'utilisateur (7) pour la sonde à ultrasons en utilisant le dispositif de suivi de sonde à ultrasons, en déterminant un emplacement (7) auquel l'utilisateur a positionné la sonde à ultrasons (5), pour marquer l'emplacement de départ, comme l'emplacement de départ; obtenir (12) un emplacement de fin défini par l'utilisateur (8) pour la sonde à ultrasons en utilisant le dispositif de suivi de sonde à ultrasons, en déterminant un emplacement (8) auquel l'utilisateur a positionné la sonde à ultrasons (5), pour marquer l'emplacement de fin, comme l'emplacement de fin, un volume entre l'emplacement de départ et l'emplacement de fin définissant ainsi le volume à imager; déterminer (13) des instructions de guidage pour spécifier un mouvement de la sonde à ultrasons de l'emplacement de départ défini par l'utilisateur à l'emplacement de fin défini par l'utilisateur, par rapport au volume à imager; et pendant un processus d'imagerie par ultrasons ultérieur du volume, surveiller, en utilisant le dispositif de suivi de sonde à ultrasons, un mouvement de la sonde à ultrasons par rapport au volume pour déterminer si l'utilisateur adhère aux instructions de guidage.

2. Procédé selon la revendication 1, dans lequel l'étape de détermination d'instructions de guidage consiste à: déterminer une durée admissible maximale du processus d'imagerie par ultrasons; déterminer une distance d'un trajet pour la sonde à ultrasons entre l'emplacement de départ défini par l'utilisateur et l'emplacement de fin défini par l'utilisateur; et déterminer une vitesse minimale de mouvement pour la sonde à ultrasons pendant le processus d'imagerie par ultrasons sur la base de la durée admissible maximale et de la distance.

3. Procédé selon la revendication 2, dans lequel l'étape de surveillance d'un mouvement de la sonde à ultrasons pendant le processus d'imagerie par ultrasons consiste à: déterminer un emplacement actuel de la sonde à ultrasons par rapport à un ou plusieurs parmi l'emplacement de départ défini par l'utilisateur et l'emplacement de fin défini par l'utilisateur; déterminer un temps actuel écoulé pendant le processus d'imagerie par ultrasons; déterminer la vitesse moyenne de mouvement pendant le processus d'imagerie par ultrasons sur la base de l'emplacement actuel de la sonde à ultrasons et du temps actuel écoulé; et déterminer si la vitesse moyenne de mouvement est supérieure ou égale à la vitesse minimale de mouvement pour déterminer si l'utilisateur adhère aux instructions de guidage.

4. Procédé selon la revendication 3, dans lequel la durée admissible maximale est basée sur un ou plusieurs parmi: une durée maximale définie par l'utilisateur; une durée maximale définie par le sujet; une durée maximale d'un processus d'imagerie par ultrasons par le système d'imagerie par ultrasons; et une fréquence de trame du système d'imagerie par ultrasons et un certain nombre d'images par ultrasons qui peuvent être stockées par le système d'imagerie par ultrasons.

5. Procédé selon l'une quelconque des revendications 1 à 4, comprenant en outre une étape de détermination d'une progression du processus d'imagerie par ultrasons sur la base du mouvement surveillé de la sonde à ultrasons pendant le processus d'imagerie par ultrasons, de l'emplacement de départ défini par l'utilisateur et de l'emplacement de fin défini par l'utilisateur, facultativement dans lequel le procédé comprend en outre une étape d'affichage de la progression du processus d'imagerie par ultrasons à un utilisateur.

6. Procédé selon l'une quelconque des revendications 1 à 5, dans lequel l'étape de détermination d'instructions de guidage comprend la détermination d'un trajet de mouvement recommandé de la sonde à ultrasons.

7. Procédé selon la revendication 6, dans lequel l'étape de surveillance d'un mouvement de la sonde à ultrasons pendant le processus d'imagerie par ultrasons consiste à: déterminer un emplacement actuel de la sonde à ultrasons; et déterminer si l'emplacement actuel se trouve sur le trajet de mouvement recommandé afin de déterminer si l'utilisateur adhère aux instructions de guidage.

8. Procédé d'imagerie par ultrasons (80) comprenant :
un procédé de guidage d'un utilisateur d'un système d'imagerie par ultrasons selon l'une quelconque des revendications 1 à 7; et un processus d'imagerie par ultrasons (81, 82) comprenant la capture d'images par ultrasons lorsque la sonde à ultrasons est déplacée de l'emplacement de départ défini par l'utilisateur à l'emplacement de fin défini par l'utilisateur.

9. Procédé d'imagerie par ultrasons selon la revendication 8, consistant en outre à:
déterminer (84) quand la sonde à ultrasons est située à l'emplacement de départ défini par l'utilisateur et à la fois les emplacements de départ et de fin définis par l'utilisateur ont été obtenus; et en réponse à la détermination que la sonde à ultrasons est à l'emplacement de départ défini par l'utilisateur et que les emplacements de départ et de fin définis par l'utilisateur ont été obtenus, mettre en œuvre (81) le processus d'imagerie par ultrasons.

**10.** Procédé d'imagerie par ultrasons selon l'une quelconque des revendications précédentes, comprenant en outre l'obtention d'un emplacement intermédiaire défini par l'utilisateur (49) pour la sonde à ultrasons en utilisant le dispositif de suivi de sonde à ultrasons, dans lequel l'étape de détermination d'instructions de guidage comprend la détermination d'instructions de guidage pour spécifier un mouvement de la sonde à ultrasons depuis l'emplacement de départ défini par l'utilisateur (47), jusqu'à l'emplacement intermédiaire (49) et jusqu'à l'emplacement de fin défini par l'utilisateur (48), par rapport au volume à imager.

**11.** Programme informatique comprenant des moyens de code pour mettre en œuvre le procédé selon l'une quelconque des revendications 1 à 10 lorsque ledit programme est exécuté sur un ordinateur d'un système d'imagerie par ultrasons.

**12.** Système de guidage de sonde à ultrasons (110) pour un système d'imagerie à ultrasons (100) présentant une sonde à ultrasons portative (104) et un dispositif de suivi de sonde à ultrasons (105) adapté pour obtenir un emplacement de la sonde à ultrasons, le système de guidage de sonde à ultrasons comprenant: une unité de définition de volume (111) adaptée pour: obtenir un emplacement de départ défini par l'utilisateur pour la sonde à ultrasons en utilisant le dispositif de suivi de sonde à ultrasons, en déterminant un emplacement (7) auquel l'utilisateur a positionné la sonde à ultrasons (5), pour marquer l'emplacement de départ, comme l'emplacement de départ; obtenir un emplacement de fin défini par l'utilisateur pour la sonde à ultrasons en utilisant le dispositif de suivi de sonde à ultrasons, en déterminant un emplacement (8) auquel l'utilisateur a positionné la sonde à ultrasons (5), pour marquer l'emplacement de fin, comme l'emplacement de fin, un volume entre l'emplacement de départ et l'emplacement de fin pour ainsi définir un volume dans lequel l'imagerie par ultrasons doit être mise en oeuvre; une unité de détermination (112) adaptée pour déterminer des instructions de guidage pour spécifier un mouvement de la sonde à ultrasons de l'emplacement de départ défini par l'utilisateur à l'emplacement de fin défini par l'utilisateur par rapport au volume dans lequel l'imagerie par ultrasons doit être mise en oeuvre; et une unité de surveillance (113) adaptée pour, pendant un processus d'imagerie par ultrasons ultérieur, surveiller, en utilisant le dispositif de suivi de sonde à ultrasons, un mouvement de la sonde à ultrasons par rapport au volume pour déterminer si l'utilisateur adhère aux instructions de guidage.

**13.** Système de guidage de sonde à ultrasons selon la revendication 12, dans lequel l'unité de détermination est adaptée pour: déterminer une durée admissible maximale du processus d'imagerie par ultrasons; déterminer une distance d'un trajet entre l'emplacement de départ défini par l'utilisateur et l'emplacement de fin défini par l'utilisateur; et déterminer une vitesse minimale de mouvement pour la sonde à ultrasons pendant le processus d'imagerie par ultrasons sur la base de la durée admissible maximale et de la distance.

**14.** Système de guidage de sonde à ultrasons selon la revendication 12 ou 13, dans lequel: l'unité de définition de volume est en outre adaptée pour obtenir un emplacement intermédiaire défini par l'utilisateur pour la sonde à ultrasons en utilisant le dispositif de suivi de sonde à ultrasons; et l'unité déterminante est adaptée pour déterminer des instructions de guidage en déterminant des instructions de guidage pour spécifier un mouvement de la sonde à ultrasons depuis l'emplacement de départ défini par l'utilisateur, à travers l'emplacement intermédiaire et jusqu'à l'emplacement de fin défini par l'utilisateur, par rapport au volume à imager.

**15.** Système d'imagerie par ultrasons (100) comprenant:
une sonde à ultrasons (104); un dispositif de suivi de sonde à ultrasons (105); un système de guidage de sonde à ultrasons (110) selon l'une quelconque des revendications 11 à 14.

FIG. 1

FIG. 2

FIG. 3

FIG. 4

FIG. 5

51  57A  58A

58B

57B

## FIG. 6

2

5

6

69

65

57B

O

58B

61

63

## FIG. 7

FIG. 8

FIG. 9

**EP 3 713 494 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017182397 A1
- US 20150366535 A1
- JP 2014121434 A
- US 2013142010 A1
- US 20090024030 A1